# EUROPÄISCHE PATENTSCHRIFT

(11) **EP 1 529 033 B1**
(45) Veröffentlichungstag und Bekanntmachung des Hinweises auf die Patenterteilung: **06.10.2010**
(21) Anmeldenummer: 03784115.2
(22) Anmeldetag: 30.07.2003
(51) Int. Cl.: C07C 263/10, C07C 263/20, C25B 1/26, B01J 23/46

(54) **INTEGRIERTES VERFAHREN ZUR HERSTELLUNG VON ISOCYANATEN**
INTEGRATED METHOD FOR PRODUCING ISOCYANATES
PROCEDE INTEGRE DE PRODUCTION D'ISOCYANATES

(30) Priorität: 02.08.2002 DE 10235476
(43) Veröffentlichungstag der Anmeldung: 11.05.2005
(73) Patentinhaber: BASF SE, 67056 Ludwigshafen (DE)
(72) Erfinder: WALSDORFF, Christian, 67059 Ludwigshafen (DE); FIENE, Martin, 67150 Niederkirchen (DE); STROEFER, Eckhard, 68163 Mannheim (DE); HARTH, Klaus;, 67317 Altleiningen (DE); JACOBS, Jan, D., Baton Rouge, LA 70810 (US); DEBERDT, Filip, 2812 Muizen (BE)
(86) Internationale Anmeldenummer: PCT/EP2003/008430
(87) Internationale Veröffentlichungsnummer: WO 2004/014845

(56) Entgegenhaltungen:
- WO-A-97/24320
- DE-A- 19 634 192

## Beschreibung

Die Erfindung betrifft ein Verfahren zur Herstellung von Isocyanaten.

Isocyanate und Isocyanatgemische werden nach bekannten Verfahren durch Phosgenierung der entsprechenden Amine hergestellt. Für Polyurethanschäume finden beispielsweise di- oder polyfunktionelle aromatische Isocyanate der Diphenylmethandiisocyanat-Reihe (MDI) Anwendung. Bedingt durch den Herstellungsprozess werden nach der Phosgenierung und der anschließenden Aufarbeitung (Abtrennung des Lösemittels; Abtrennen von monomerem MDI) oft dunkel gefärbte Produkte erhalten, die wiederum gelblich verfärbte Polyurethanschäume oder andere, ebenfalls verfärbte Polyurethan-Materialien ergeben. Dies ist unerwünscht, da eine solche Färbung den visuellen Gesamteindruck beeinträchtigt und geringfügige Inhomogenitäten hervortreten lässt, z. B. als Schlieren in den erhaltenen Schäumen. Helle Isocyanate, bzw. Isocyanate, die eine reduzierte Menge an farbgebenden Komponenten enthalten, werden deshalb als Rohstoffe bevorzugt.

Die Gegenwart von Brom und Iod wirkt sich ferner durch Nebenproduktbildung ausbeutemindernd aus. Die Abtrennung der Nebenprodukte bringt einen erhöhten trennaufwand mit sich. Bei der Abtrennung der Nebenprodukte geht zusätzliches Wertprodukt verloren.

In WO 01/00569 ist ein Verfahren zur Herstellung von Isocyanaten, die keine oder nur geringe Mengen an farbgebenden Komponenten aufweisen, offenbart, das ohne weitere Vor- oder Nachbehandlungsschritte zu hellen Isocyanaten führt, die zur Herstellung von Polyurethanen mit keiner oder nur geringer Färbung geeignet sind. Dieses Verfahren ist dadurch gekennzeichnet, dass bei der Herstellung der Isocyanate Phosgen eingesetzt wird, das weniger als 50 ppm an Brom oder bromhaltigen Verbindungen oder Iod oder iodhaltigen Verbindungen aufweist.

Technisch wird Chlor aus Steinsalz, Meersalz oder Kalisalz hergestellt. Üblicherweise wird dabei Chlor zusammen mit Natrium oder Natronlauge als Koppelprodukt durch Elektrolyse einer Steinsalzlösung produziert. Analog wird Kaliumchlorid zur Produktion von Chlor neben Kalium oder Kalilauge eingesetzt. Die bei der Elektrolyse eingesetzten Salze enthalten üblicherweise Brom- und Iodverbindungen in Mengen zwischen 30 und 3000 ppm, die bei der Elektrolyse Brom bzw. Iod bilden.

Nachteilig an dem oben beschriebenen Verfahren ist der hohe Reinigungsaufwand, der betrieben werden muss, um den Brom- bzw. Iodgehalt in dem zur Phosgensynthese eingesetzten Chlor derart weit abzusenken, dass das erhaltene, bei der Isocyanatherstellung eingesetzte Phosgen den geforderten geringen Gehalt an Brom, Iod, bromhaltigen oder iodhaltigen Verbindungen aufweist.

Aus EP-A 0 876 335 ist ein Verfahren zur Herstellung von Isocyanaten aus Phosgen und Aminen bekannt, bei dem der bei der Isocyantherstellung anfallende Chlorwasserstoff elektrolytisch zu Chlor oxidiert wird. Das erhaltene Chlor wird in die Phosgensynthese rezykliert. Allerdings ist die Elektrolyse von Chlorwasserstoff mit hohen Elektrizitätskosten verbunden. Auch fällt bei diesem Verfahren als Koppelprodukt Wasserstoff an. Dieser ist unter Sicherheitsaspekten bedenklich. In der genannten Schrift wird darauf abgehoben, dass der elektrolytisch erzeugte Wasserstoff zur Herstellung von Aminen aus den entsprechenden Nitroverbindungen eingesetzt wird. Dieser Vorteil entfällt jedoch, falls die Isocyanatherstellung nicht bis zur Herstellung der Amine aus den entsprechenden Nitroverbindungen rückintegriert ist. Ohnehin reicht der bei der Chlorwasserstoff-Elektrolyse gebildete Wasserstoff nicht zur Reduktion der Nitroverbindungen aus. Ferner ist nachteilig, dass schon geringe Spuren organischer Verbindungen, beispielsweise Lösemittelreste aus der Isocyanatherstellung, in der empfindlichen Chlorwasserstoff-Elektrolyse störend sind, so dass der eingesetzte Chlorwasserstoff sehr rein sein muss.

Aufgabe der Erfindung ist es, ein effizientes Verfahren zur Herstellung von hellen Isocyanaten bereitzustellen, das ohne weitere Vor- oder Nachbehandlungsschritte zur Aufhellung der erhaltenen Isocyanate auskommt, und bei dem der Aufwand zur Reinigung der eingesetzten Rohstoffe minimal ist.

Gelöst wird die Aufgabe durch ein Verfahren zur Herstellung von hellen organischen Isocyanaten mit den Schritten
(a) Bereitstellung einer ersten Teilmenge Chlor, wobei das Chlor der ersten Teilmenge einen Gehalt an freiem oder gebundenem Brom und Iod von < 400 ppm aufweist;
(b) Bereitstellung einer zweiten Teilmenge Chlor;
(c) Umsetzung der ersten und der zweiten Teilmenge Chlor mit Kohlenmonoxid zu Phosgen;
(d) Umsetzung des Phosgens aus Schritt (c) mit einem oder mehreren primären Aminen zu den entsprechenden Isocyanaten und Chlorwasserstoff;
(e) Abtrennung und gegebenenfalls Reinigung der in Schritt (d) gebildeten Isocyanate;
(f) Abtrennung und gegebenenfalls Reinigung des in Schritt (d) gebildeten Chlorwasserstoffs;
(g) katalytische Oxidation zumindest eines Teils des in Schritt (f) abgetrennten Chlorwasserstoffs mit Sauerstoff zu Chlor;
(h) Abtrennung des in Schritt (g) gebildeten Chlors und Einsatz zumindest einer Teilmenge des abgetrennten Chlors als zweite Teilmenge Chlor in Schritt (b).

In einem Schritt (a) wird eine erste Teilmenge Chlor bereitgestellt. Das Chlor der ersten Teilmenge weist einen Gehalt an freiem oder gebundenem Brom und Iod von < 400 ppm auf. 1 ppm Brom oder Iod bedeutet dabei 1 Atom Brom oder Iod pro 1 000 000 Halogenatomen. Die Obergrenze von 400 ppm bezieht sich dabei auf die Summe beider Elemente.

Brom und Iod können in dem Chlor in molekularer (freier) Form als Br₂ bzw. I₂ oder aber auch in gebundener Form, beispielsweise als BrCl und ICl vorliegen.

Verfahren zur Herstellung von entsprechendem Chlor mit einem niedrigen Gehalt an Brom und Iod sind in der Fachwelt bekannt. Grundsätzlich kann im Rahmen der vorliegenden Erfindung jedes Chlor eingesetzt werden, das die o. g. Spezifikation erfüllt, also weniger als etwa 400 ppm Brom und Iod aufweist. So kann beispielsweise Chlor eingesetzt werden, das durch Elektrolyse-Verfahren oder Oxidation von Chlorwasserstoff, z.B. nach dem Deacon-Prozess, hergestellt wurde, sofern auch der eingesetzte Chlorwasserstoff einen ausreichend niedrigen Brom- und Iodgehalt aufweist.

In einer Ausführungsform der Erfindung wird das Chlor der ersten Teilmenge durch Elektrolyte einer Chloridionen enthaltenden Lösung erzeugt. Im allgemeinen ist dies eine wässrige Steinsalzlösung, eine wässrige Kalilsalzlösung oder wässrige Chlorwasserstoffsäure (Salzsäure).

So können entsprechend geeignete Ausgangsstoffe für die Chlorsynthese eingesetzt werden, die selbst wenig Brom und Iod enthalten, z. B. brom- und iodarmes Salz oder brom- und iodarme Salzsäure. Entsprechende brom- und iodarme Salze mit einem Brom- und Iodgehalt von insgesamt < 400 ppm wird beispielsweise bei Heilbronn, DE, abgebaut.

Die Herstellung von Chlor mit einem besonders niedrigen Gehalt an Brom kann auch, wie in der US 3,660,261 beschrieben, durch oxidative Behandlung des für die Elektrolyse verwendeten Salzes erfolgen.

In einer weiteren Ausführungsform der Erfindung wird das Chlor der ersten Teilmenge nach seiner Herstellung einer Abreicherungsstufe unterzogen, in der es an Brom und/oder Iod abgereichert wird.

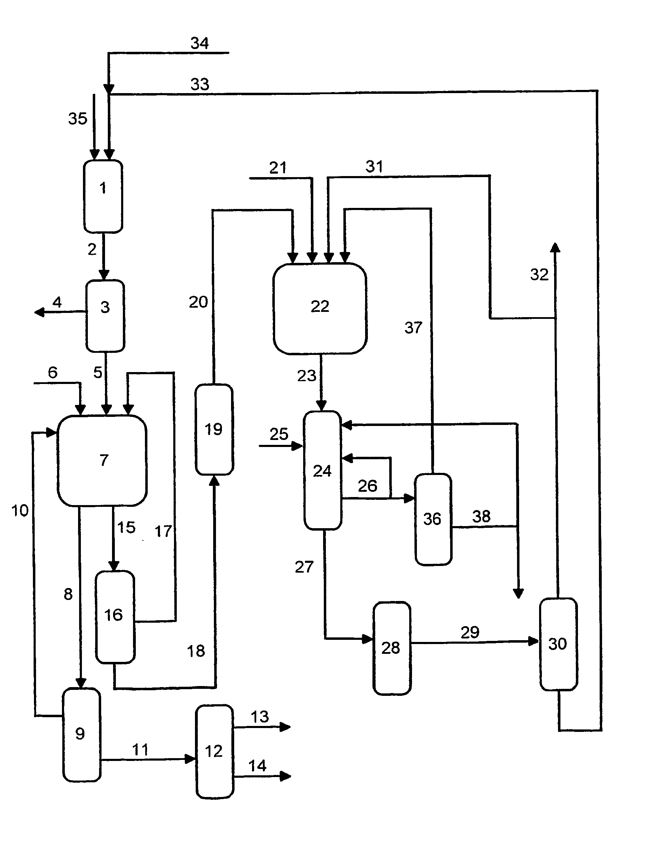

Eine Möglichkeit der Abreicherung von Brom in bromhaltigem Chlor ist in Ullmann's Encyclopedia of Industrial Chemistry, 5. Auflage, Band A6, S. 463 und Abbildung 70 auf S. 465 beschrieben: Bei der Wäsche von gasförmigem bromreichem Chlor mit flüssigem bromarmen Chlor im Gegenstrom verarmt das zu reinigende Chlorgas an Brom und das flüssige Chlor reichert sich mit Brom an. Zur Inbetriebnahme einer entsprechenden Anlage ist die Bereitstellung einer ausreichenden Menge an bromarmem flüssigem Chlor notwendig, anschließend kann ein Teilstrom des gewonnenen bromärmeren Chlors verflüssigt und zur Wäsche des bromreicheren Chlors benutzt werden. Dieses Verfahren wird in Kolonnen mit üblichen trennwirksamen Einbauten wie Böden, Füllkörper oder Packungen durchgeführt. Der erreichte Abreicherungsgrad an Brom bzw. Iod hängt in der für Absorptions- und Destillationsverfahren üblichen Weise von Systemdruck, den Strommengen, den Konzentrationen und den verwendeten Einbauten ab. Die Auslegung der Kolonne anhand des gewünschten Brom-Abreicherungsgrads ist daher eine reine Routineaufgabe.

Alternativ dazu ist die Entfernung von Brom oder Iod aus Chlor mittels Destillation, selektiver Kondensation des Broms oder Iods im Chlorstrom oder durch Reaktionen mit Stoffen, die selektiv mit Brom und/oder Iod reagieren, möglich, wie es beispielsweise in der JP 0075319 beschrieben wird. Geeignete Verfahren sind auch in DE-OS 18 00 844, DE-AS 12 55 643 oder in DE-A1 197 26 530 beschrieben.

Bevorzugt enthält das als erste Teilmenge eingesetzte Chlor weniger als 200 ppm Brom und Iod, besonders bevorzugt weniger als 50 ppm Brom und Iod.

In einem Schritt (b) wird eine zweite Teilmenge Chlor bereitgestellt. Zu Beginn der Durchführung des erfindungsgemäßen Verfahrens, also während der "Anfahrphase", aber auch bei Änderung der Produktionsmenge während der Durchführung des Verfahren oder während der "Abfahrphase", kann das Chlor der zweiten Teilmenge den gleichen oder sogar einen deutlich höheren Gehalt an Brom und Iod aufweisen, vorzugsweise wird es den gleichen oder einen niedrigeren Gehalt an Brom und Iod aufweisen, insbesondere werden das Chlor der ersten und zweiten Teilmenge während der Startphase aus der gleichen Quelle stammen. Ist das erfindungsgemäße Verfahren in seiner Durchführung über die Startphase hinaus gelangt und steht eine ausreichende Menge Chlor aus dem Abtrennschritt (h) als zweite Teilmenge zur Verfügung, wird diese Teilmenge in jedem Fall einen deutlich niedrigeren Brom- und Iodgehalt als das Chlor der ersten Teilmenge aufweisen, da das in Schritt (h) wiedergewonnene Chlor bereits die Schritte der Phosgensynthese (c) und der Isocyanatherstellung (d) durchlaufen hat. Das in dem eingesetzten Chlor bzw. in dem daraus hergestellten Phosgen enthaltene Brom und Iod reagiert während der Isocyanatherstellung (d) unter Kern- und Seitenkettenbromierungen bzw. -iodierungen mit den Aminen und Isocyanaten und verbleiben in diesen gebunden. Auf diese Weise wird Brom bzw. Iod aus dem eingesetzten Chlor "herausgewaschen". Es stellt sich ein stationärer Zustand ein, der dadurch gekennzeichnet ist, dass das in Schritt (h) wiedergewonnene und als zweite Teilmenge eingesetzte Chlor einen sehr viel geringeren Brom- und Iodgehalt aufweist als das Chlor der ersten Teilmenge oder sogar nahezu frei von Brom oder Iod ist, so dass das in Schritt (c) eingesetzte Chlor insgesamt einen sehr viel niedrigeren Brom- oder Iodgehalt als das dem Verfahren zugeführte Chlor der ersten Teilmenge aufweist. Dabei ist nur das während der Startphase der Durchführung des erfindungsgemäßen Verfahrens erhaltene Isocyanat mit relativ hohen Brom- und Iodgehalten "kontaminiert", während das in einer späteren Phase hergestellte Isocyanat nur noch mit den sehr viel niedrigeren stationären Brom- und Iodgehalten in Berührung gekommen ist. Die Anfangskontamination lässt sich mindern, indem während der Startphase besonders reines Chlor als Chlor der ersten Teilmenge eingesetzt wird.

Unter stationären Bedingungen wird vorzugsweise im wesentlichen die Gesamtmenge des in Schritt (h) abgetrennten Chlors als zweite Teilmenge Chlor in Schritt (b) eingesetzt, und wird die erste Teilmenge Chlor so bemessen, dass die Summe aus erster und zweiter Teilmenge konstant bleibt. In anderen Worten werden die über die Schritte (c) bis (h) erfolgten Verluste an Chlor durch die erste Teilmenge lediglich kompensiert.

Üblicherweise beträgt der Anteil der zweiten Teilmenge Chlor an der Summe aus erster und zweiter Teilmenge Chlor mindestens 70%.

Beträgt der Anteil der (rückgeführten) zweiten Teilmenge Chlor an der Summe aus erster und zweiter Teilmenge Chlor beispielsweise ca. 80 %, so werden bei gegebenem Gehalt an Brom und Iod in dem eingesetzten Chlor nur ca. 1/5 der Brom- und Iodmenge eingebracht, die ohne die Chlor-Rezyklierung eingebracht würde. Somit können in der ersten Teilmenge Chlor noch verhältnismäßig hohe Gehalte an Brom oder Iod toleriert werden, und werden dennoch auch ohne weitere Vor- oder Nachbehandlungsschritte helle Isocyanate erhalten. Damit kann der mit der Abreicherung von Brom und Iod in dem eingesetzten Chlor verbundene Aufwand auch ganz entfallen. Umgekehrt werden mit brom- und iodarmem Chlor nach dem erfindungsgemäßen Verfahren noch sehr viel höhere Reinheitsgrade des hergestellten Isocyanats erzielt, als dies ohnehin schon der Fall wäre.

In einer Stufe (c) werden die erste und die zweite Teilmenge Chlor mit Kohlenmonoxid zu Phosgen umgesetzt. Verfahren zur Herstellung von Phosgen sind in Ullmanns Enzyklopädie der industriellen Chemie, 3. Aufl., Bd. 13, Seite 494 - 500 beschrieben. So kann Phosgen durch Überleiten von Kohlenmonoxid und Chlor über Aktivkohle erhalten werden.

In einer Stufe (d) wird Phosgen mit einem oder mehreren Aminen zu den entsprechenden Isocyanaten und Chlorwasserstoff umgesetzt. Diese Reaktion wird auch als Phosgenierung der Amine bezeichnet. Die eingesetzten Amine weisen mindestens eine, bevorzugt zwei, gegebenenfalls auch drei oder mehr primäre Aminogruppen auf.

Die im Rahmen des erfindungsgemäßen Verfahrens stattfindende Isocyanatherstellung wird in einer dem Fachmann bekannten Weise durch Umsetzung eines Amins oder eines Gemischs aus zwei oder mehr Aminen mit Phosgen in überstöchiometrischer Menge durchgeführt. Anwendbar sind grundsätzlich alle Verfahren, bei denen ein primäres Amin oder ein Gemisch aus zwei oder mehr primären Aminen mit einer oder mehreren primären Aminogruppen mit Phosgen unter Bildung einer oder mehrerer Isocyanate mit einer oder mehrerer Isocyanatgruppen umgesetzt wird.

In einer bevorzugten Ausführungsform der Erfindung wird die Phosgenierung des oder der Amine in einem Lösemittel oder Lösungsmittelgemisch durchgeführt. Als Lösemittel können alle für die Herstellung von Isocyanaten geeigneten Lösemittel eingesetzt werden. Vorzugsweise sind dies inerte aromatische, aliphatische oder alicyclische Kohlenwasserstoffe oder deren halogenierte Derivate. Beispiele für solche Lösemittel sind aromatische Verbindungen wie Mono- oder Dichlorbenzol, beispielsweise o-Dichlorbenzol, Toluol, Xylole, Naphthalinderivate wie Tetralin oder Decalin, Alkane mit etwa 5 bis etwa 12 C-Atomen wie Hexan, Heptan, Octan, Nonan oder Decan, Cycloalkane wie Cyclohexan, weitgehend inerte Ester und Ether wie Ethyl- oder Butylacetat, Tetrahydrofuran, Dioxan oder Diphenylether. Es kann auch ein Teilstrom des produzierten Isocyanats als Lösungsmittel oder Lösungsmittelbestandteil zurückgeführt werden.

Als Amine eignen sich prinzipiell alle primären Amine, die in geeigneter Weise mit Phosgen zu Isocyanaten reagieren können. Geeignet sind prinzipiell alle linearen oder verzweigten, gesättigten oder ungesättigten aliphatischen oder cycloaliphatischen oder aromatischen primären Mono- oder Polyamine, die mit Phosgen zu Isocyanaten umgesetzt werden können. Beispiele für geeignete Amine sind 1,3-Propylendiamin, 1,4-Butylendiamin, 1,5-Pentamethylendiamin, 1,6-Hexamethylendiamin und die entsprechenden höheren Homologen dieser Reihe, Isophorondiamin (IPDA), Cyclohexylendiamin, Cyclohexylamin, Anilin, Phenylendiamin, p-Toluidin, 1,5-Naphtylendiamin, 2,4- oder 2,6-Toluylendiamin oder deren Gemische, 4,4'-, 2,4'- oder 2,2'-Diphenylmethandiamin oder deren Gemische, sowie höhermolekulare isomere, oligomere oder polymere Derivate der obengenannten Amine und Polyamine.

In einer bevorzugten Ausführungsform der vorliegenden Erfindung werden als Amine die isomeren primären Diphenylmethandiamine (MDA) bzw. deren oligomere oder polymere Derivate eingesetzt, also die Amine der Diphenylmethandiamin-Reihe. Diphenylmethandiamin, dessen Oligomere oder Polymere werden beispielsweise durch Kondensation von Anilin mit Formaldehyd erhalten. Auch solche Oligo- oder Polyamine oder deren Gemische werden im Rahmen einer bevorzugten Ausführungsform der Erfindung eingesetzt. Bevorzugte Amine sind weiterhin Hexamethylendiamin, Toluylendiamin und Isophorondiamin.

Die Umsetzung (d) des brom- und iodarmen Phosgens mit den obengenannten Aminen kann kontinuierlich oder diskontinuierlich in einer oder mehreren Stufen erfolgen. Wird eine einstufige Umsetzung durchgeführt, so erfolgt diese Umsetzung vorzugsweise bei etwa 40 bis 200 °C, beispielsweise bei etwa 90 bis 180 °C.

In einer bevorzugten Ausführungsform der Erfindung wird die Umsetzung (d) zweistufig durchgeführt. Hierbei wird in einer ersten Stufe, die auch als Kaltphosgenierung bezeichnet wird, die Umsetzung des Phosgens mit dem oder den Aminen bei einer Temperatur von 0 bis 160 °C, beispielsweise von 20 bis 130 °C durchgeführt, wobei für die Reaktion zwischen Amin und Phosgen eine Zeitspanne von etwa 0,5 min bis 2 h eingeräumt wird. Anschließend wird in einer zweiten Stufe, die auch als Heißphosgenierung bezeichnet wird, die Temperatur innerhalb einer Zeitspanne von im allgemeinen etwa 1 min bis 5 h, beispielsweise innerhalb von etwa 1 min bis 3 h, auf 60 bis 190 °C, insbesondere 70 bis 170 °C, erhöht.

Während der Umsetzung (d) kann in einer weiteren Ausführungsform der Erfindung erhöhter Druck angelegt werden, im allgemeinen bis zu 100 bar oder weniger, vorzugsweise 1 bar bis etwa 50 bar, besonders bevorzugt 2 bar bis 25 bar, insbesondere 3 bar bis 12 bar. In einer weiteren Ausführungsform der Erfindung wird bei etwa 1 bar (Umgebungsdruck) gearbeitet. In einer weiteren Ausführungsform wird bei gegenüber dem Umgebungsdruck reduziertem Druck gearbeitet.

In einem Schritt (e) werden die gebildeten Isocyanate abgetrennt und gegebenenfalls gereinigt.

Überschüssiges Phosgen kann im Anschluss an die Umsetzung bei einer Temperatur von 50 bis 180 °C entfernt werden. Die Entfernung des Lösemittels erfolgt vorzugsweise unter vermindertem Druck, beispielsweise bei einem Druck von 500 mbar oder weniger, bevorzugt von 100 mbar oder weniger. Im Allgemeinen werden dabei die verschiedenen Lösemittel-Komponenten in der Reihe der Siedepunkte abgetrennt, wobei auch die Abtrennung von Gemischen der verschiedenen Komponenten in einer einzigen Verfahrensstufe möglich ist. Anschließend kann das erhaltene Isocyanat fraktioniert werden.

In einem Schritt (f) wird der Chlorwasserstoff abgetrennt und gegebenenfalls gereinigt. Chlorwasserstoff fällt bei der Umsetzung (d) von Phosgen mit Amin üblicherweise gasförmig im Gemisch mit Phosgen und typischer Weise geringen Mengen weiterer Gase wie Kohlenmonoxid, Kohlendioxid, Stickstoff und Spuren von in der Isocyanatherstellung eingesetzten Lösemitteln an. Phosgen und schwersiedende Nebenbestandteile können durch Destillation abgetrennt werden. Es wird ein im wesentlichen Chlorwasserstoff enthaltender Strom erhalten. Darin enthaltene Spuren organischer Verbindungen wie Phosgen und Lösemittelreste können in einer nachgeschalteten Reinigungsstufe durch Absorption, Adsorption, Destillation oder Extraktion entfernt werden. Zur Reinigung kann der Chlorwasserstoffstrom auch in Wasser oder verdünnter Salzsäure absorbiert werden und in einem weiteren Schritt nach Abtrennung flüchtiger Bestandteile wieder desorbiert werden. Lösemittelreste können auch durch katalytische Verbrennung in dem Chlorwasserstoffstrom entfernt werden. Der gegebenenfalls so gereinigte Chlorwasserstoffstrom wird der katalytischen Chlorwasserstoff-Oxidation zugeführt.

In einer Ausführungsform des erfindungsgemäßen Verfahrens erfolgt die Reinigung des Chlorwasserstoff enthaltenden Stroms durch Überleiten über ein Reinigungsbett und Absorption von darin enthaltenen Lösemittelresten an dem Reinigungsbett.

Das Reinigungsbett besteht aus geeigneten Absorbentien, vorzugsweise in stückiger Form wie Kugeln, Extrudate oder Tabletten. Geeignete Materialien, die als Absorbentien in Frage kommen, sind beispielsweise Aktivkohle, Aluminiumoxid, Titanoxid, Siliziumdioxid, Eisenoxid, Zeolithe und Molsiebe. Geeignete Materialien können auch Metalloxide oder Metallhalogenide, wie Kupfer- oder Rutheniumoxide oder -halogenide bzw. deren Gemische, auf einem Träger aus einem feuerfesten anorganischen Material wie Aluminiumoxid, Titanoxid oder Siliziumdioxid enthalten. Bevorzugte Absorbentien sind Aluminiumoxid, Aktivkohle und Tonerden.

In einer weitem Ausführungsform des erfindungsgemäßen Verfahrens erfolgt die Reinigung des Chlorwasserstoff enthaltenden Stroms durch katalytische Verbrennung der darin enthaltenen Lösemittelreste. Dazu wird dem Chlorwasserstoff enthaltenden Strom Sauerstoff oder ein Sauerstoff enthaltender Gasstrom, beispielsweise Luft, mit Sauerstoff angereicherte Luft, technischer oder reiner Sauerstoff, zugemischt und dieser Strom über ein Katalysatorfestbett aus Oxidationskatalysator geleitet. Geeignete Katalysatoren enthalten beispielsweise Aluminiumoxid, Magnesiumoxid, Eisenoxid, Titandioxid, Zirkondioxid oder deren Gemische. Die katalytische Verbrennung der Lösemittelreste (Kohlenwasserstoffe und/oder Chlorkohlenwasserstoffe) an den genannten Katalysatoren kann bereits einen Teilumsatz des enthaltenen Chlorwasserstoffs zum Chlor bewirken. Dieser Teilumsatz kann beispielsweise bis zu 40%, bevorzugt bis zu 20 %, beispielsweise ca. 5 bis 20% betragen.

Die Durchführung der katalytischen Verbrennung als Reinigungsstufe kann auch als erste Stufe einer zweistufigen katalytischen Chlorwasserstoff-Oxidation betrachtet werden, wobei die erste Stufe an den oben genannten Katalysatoren bis zu einem Teilumsatz und die zweite Stufe als Schritt (g) an den unten beschriebenen Ruthenium enthaltenden Katalysatoren bis zum Vollumsatz, beispielsweise einem Umsatz von mindestens 70%, bezogen auf die erste und die zweite Stufe, durchgeführt wird. Die erste Stufe, die an preiswerten, relativ unempfindlichen Katalysatoren durchgeführt wird, bewirkt eine Oxidation der zu Koksablagerungen führenden Lösemittespuren zu Kohlendioxid. Dadurch ist der in der zweiten Stufe eingesetzte teure Rutheniumkatalysator vor koksbildenden Verunreinigungen geschützt.

In einem Schritt (g) erfolgt die katalytische Oxidation des in Schritt (f) abgetrennten Chlorwasserstoffs mit Sauerstoff zu Chlor.

Dazu werden der gegebenenfalls gereinigte Chlorwasserstoffstrom, gegebenenfalls ein Chlorwasserstoff enthaltender Rückstrom, ein Sauerstoff enthaltender Strom und gegebenenfalls ein Sauerstoff enthaltender Rückstrom in eine Oxidationszone eingespeist und Chlorwasserstoff in Gegenwart eines Katalysators teilweise zu Chlor oxidiert, wobei ein Produktgasstrom erhalten wird, der Chlor, nicht umgesetzten Sauerstoff, nicht umgesetzten Chlorwasserstoff und Wasserdampf enthält.

In dem auch als Deacon-Prozess bekannten katalytischen Verfahren wird Chlorwasserstoff mit Sauerstoff in einer exothermen Gleichgewichtsreaktion zu Chlor oxidiert, wobei Wasserdampf anfällt. Übliche Reaktionstemperaturen liegen zwischen 150 und 500°C, übliche Reaktionsdrucke liegen zwischen 1 und 25 bar. Da es sich um eine Gleichgewichtsreaktion handelt, ist es zweckmäßig, bei möglichst niedrigen Temperaturen zu arbeiten, bei denen der Katalysator noch eine ausreichende Aktivität aufweist. Ferner ist es zweckmäßig, Sauerstoff in überstöchiometrischen Mengen einzusetzen. Üblich ist beispielsweise ein zwei- bis vierfacher Sauerstoff-Überschuss. Da keine Selektivitätsverluste zu befürchten sind, kann es wirtschaftlich vorteilhaft sein, bei relativ hohen Drücken und dementsprechend bei gegenüber Normaldruck längeren Verweilzeiten zu arbeiten.

Geeignete Katalysatoren enthalten Rutheniumoxid, Rutheniumchlorid oder andere Rutheniumverbindungen auf Siliziumdioxid, Aluminiumoxid, Titandioxid oder Zirkondioxid als Träger. Geeignete Katalysatoren können beispielsweise durch Aufbringen von Rutheniumchlorid auf den Träger und anschließendes Trocknen oder Trocknen und Calcinieren erhalten werden. Geeignete Katalysatoren können ergänzend zu oder an Stelle einer Rutheniumverbindung auch Verbindungen anderer Edelmetalle, beispielsweise Gold, Palladium, Platin, Osmium, Iridium, Silber, Kupfer oder Rhenium enthalten. Geeignete Katalysatoren können ferner Chrom(III)oxid enthalten.

Übliche Reaktionsapparate, in denen die katalytische Chlorwasserstoff-Oxidation durchgeführt wird, sind ein Festbett- oder Wirbelbettreaktor. Die Chlorwasserstoff-Oxidation kann mehrstufig durchgeführt werden.

Die katalytische Chlorwasserstoff-Oxidation kann adiabat oder bevorzugt isotherm oder annähernd isotherm, diskontinuierlich, bevorzugt kontinuierlich als Fließ- oder Festbettverfahren, bevorzugt als Festbettverfahren, besonders bevorzugt in Rohrbündelreaktoren an Heterogenkatalysatoren bei Reaktortemperaturen von 180 bis 500°C, bevorzugt 200 bis 400°C, besonders bevorzugt 220 bis 350°C und einem Druck von 1 bis 25 bar, bevorzugt 1,2 bis 20 bar, besonders bevorzugt 1,5 bis 17 bar und insbesondere 2,0 bis 15 bar durchgeführt werden.

Bei der isothermen oder annähernd isothermen Fahrweise können auch mehrere, also 2 bis 10, bevorzugt 2 bis 6, besonders bevorzugt 2 bis 5, insbesondere 2 bis 3 in Reihe geschaltete Reaktoren mit zusätzlicher Zwischenkühlung eingesetzt werden. Der Sauerstoff kann entweder vollständig zusammen mit dem Chlorwasserstoff vor dem ersten Reaktor oder über die verschiedenen Reaktoren verteilt zugegeben werden. Diese Reihenschaltung einzelner Reaktoren kann auch in einem Apparat zusammengeführt werden.

Eine bevorzugte Ausführungsform besteht darin, dass man eine strukturierte Katalysatorschüttung einsetzt, bei der die Katalysatoraktivität in Strömungsrichtung ansteigt. Eine solche Strukturierung der Katalysatorschüttung kann durch unterschiedliche Tränkung der Katalysatorträger mit Aktivmasse oder durch unterschiedliche Verdünnung des Katalysators mit einem Inertmaterial erfolgen. Als Inertmaterial können beispielsweise Ringe, Zylinder oder Kugeln aus Titandioxid, Zirkondioxid oder deren Gemischen, Aluminiumoxid, Steatit, Keramik, Glas, Graphit oder Edelstahl eingesetzt werden. Beim bevorzugten Einsatz von Katalysatorformkörpern sollte das Inertmaterial bevorzugt ähnliche äußeren Abmessungen haben.

Als Katalysatorformkörper eignen sich beliebige Formen, bevorzugt sind Tabletten, Ringe, Zylinder, Sterne, Wagenräder oder Kugeln, besonders bevorzugt sind Ringe, Zylinder oder Sternstränge.

Als Heterogenkatalysatoren eignen sich insbesondere Rutheniumverbindungen oder Kupferverbindungen auf Trägermaterialen, die auch dotiert sein können, bevorzugt sind gegebenenfalls dotierte Rutheniumkatalysatoren. Als Trägermaterialen eignen sich beispielsweise Siliciumdioxid, Graphit, Titandioxid mit Rutil- oder Anatas-Struktur, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische.

Die Kupfer- bzw. die Rutheniumträgerkatalysatoren können beispielsweise durch Tränkung des Trägermaterials mit wässrigen Lösungen von CuCl₂ bzw. RuCl₃ und gegebenenfalls eines Promotors zur Dotierung, bevorzugt in Form ihrer Chloride, erhalten werden. Die Formgebung des Katalysators kann nach oder bevorzugt vor der Tränkung des Trägermaterials erfolgen.

Zur Dotierung eignen sich als Promotoren Alkalimetalle wie Lithium, Natrium, Kalium, Rubidium und Cäsium, bevorzugt Lithium, Natrium und Kalium, besonders bevorzugt Kalium, Erdalkalimetalle wie Magnesium, Calcium, Strontium und Barium, bevorzugt Magnesium und Calcium, besonders bevorzugt Magnesium, Seltenerdmetalle wie Scandium, Yttrium, Lanthan, Cer, Praseodym und Neodym, bevorzugt Scandium, Yttrium, Lanthan und Cer, besonders bevorzugt Lanthan und Cer, oder deren Gemische.

Die Formkörper können anschließend bei Temperaturen von 100 bis 400°C, bevorzugt 100 bis 300°C beispielsweise unter einer Stickstoff-, Argon- oder Luftatmosphäre getrocknet und gegebenenfalls calciniert werden. Bevorzugt werden die Formkörper zunächst bei 100 bis 150°C, getrocknet und anschließend bei 200 bis 400°C calciniert.

Der Umsatz an Chlorwasserstoff im einfachen Durchgang kann auf 15 bis 90 %, bevorzugt 40 bis 85 %, besonders bevorzugt 50 bis 70 % begrenzt werden. Nicht umgesetzter Chlorwasserstoff kann nach Abtrennung teilweise oder vollständig in die katalytische Chlorwasserstoff-Oxidation zurückgeführt werden. Das Volumenverhältnis von Chlorwasserstoff zu Sauerstoff am Reaktoreintritt liegt in der Regel zwischen 1:1 1 und 20:1, bevorzugt zwischen 2:1 und 8:1, besonders bevorzugt zwischen 2:1 und 5:1.

Die katalytische Chlorwasserstoff-Oxidation weist gegenüber der Erzeugung von Chlor durch Chlorwasserstoff-Elektrolyse den Vorteil auf, dass keine teure elektrische Energie benötigt wird, dass kein unter Sicherheitsaspekten bedenkliche Wasserstoff als Koppelprodukt anfällt und dass der zugeführte Chlorwasserstoff nicht vollständig rein sein muss.

Die Reaktionswärme der katalytischen Chlorwasserstoff-Oxidation kann in vorteilhafter Weise zur Erzeugung von Hochdruck-Wasserdampf genutzt werden. Dieser kann zum Betrieb des Phosgenierungsreaktors und der Isocyanat-Destillationskolonnen genutzt werden.

In einem Schritt (h) wird das gebildete Chlor abgetrennt. Der Abtrennschritt umfasst üblicherweise mehrere Stufen, nämlich die Abtrennung und gegebenenfalls Rückführung von nicht umgesetztem Chlorwasserstoff aus dem Produktgasstrom der katalytischen Chlorwasserstoff-Oxidation, die Trocknung des erhaltenen, im wesentlichen Chlor und Sauerstoff enthaltenden Stroms sowie die Abtrennung von Chlor aus dem getrockneten Strom.

Die Abtrennung von nicht umgesetztem Chlorwasserstoff und von gebildetem Wasserdampf kann durch Auskondensieren von wässriger Salzsäure aus dem Produktgasstrom der Chlorwasserstoffoxidation durch Abkühlung erfolgen. Chlorwasserstoff kann auch in verdünnter Salzsäure oder Wasser absorbiert werden.

In einer Ausführungsform der Erfindung wird die Abtrennung von Chlorwasserstoff wie nachstehend beschrieben durchgeführt. In einer Absorptionsstufe wird der Produktgasstrom der Chlorwasserstoffoxidation in einer Absorptionszone mit verdünnter Salzsäure oder Wasser der Konzentration c1 in Kontakt gebracht und Chlorwasserstoff in der verdünnten Salzsäure absorbiert, wobei eine Salzsäure der Konzentration c2 und ein Gasstrom, der Chlor und Sauerstoff enthält, erhalten wird. In einer Desorptionsstufe wird der absorbierte Chlorwasserstoff aus der Salzsäure der Konzentration c2 in einer Desorptionszone wieder freigesetzt. Der freigesetzte Chlorwasserstoff kann zumindest teilweise, vorzugsweise vollständig, als Chlorwasserstoff enthaltender Rückstrom in die Oxidationszone zurückgeführt werden, wo aus dem rückgeführten Chlorwasserstoff weiteres Chlor gewonnen wird. Dabei kann eine verdünnte Salzsäure der Konzentration c1 als Absorptionsmittel zurückgewonnen werden, die in die Absorptionszone zurückgeführt wird. Dabei kann auch eine Teilmenge der verdünnten Salzsäure der Konzentration c1 aus dem Verfahren ausgeschleust werden. Es kann auch, wie in der EP-A 1 099 666 beschrieben, nach Abtrennung eines Teils des Wassers in einer Niederdruckkolonne eine aufkonzentrierte Salzsäure in die Chlorwasserstoff-Desorptionszone zurückgeführt werden.

Als Absorptionsmittel geeignet ist Wasser und jede verdünnte Salzsäure, die nicht an Chlorwasserstoff gesättigt ist. Üblicher Weise wird ihre Konzentration c1 bis zu 30 Gew.-% Chlorwasserstoff, beispielsweise ca. 15 bis 20 Gew.-% betragen. Die Absorptionstemperatur beträgt üblicherweise von 0 bis 150 °C, vorzugsweise von 30 bis 100°C, der Absorptionsdruck beträgt üblicherweise von 0,5 bis 20 bar, vorzugsweise von 1 bis 10 bar. Die Desorption wird vorzugsweise in einer Desorptionskolonne durchgeführt. Der Desorptionsdruck beträgt üblicherweise von 0,3 bis 10 bar, vorzugsweise von 0,5 bis 5 bar. Die Aufarbeitung der Produktströme der Chlorwasserstoff-Oxidation und die Abtrennung von Chlorwasserstoff kann auch wie in EP-A 0 765 838 erfolgen.

Es wird ein Gasstrom erhalten, der Chlor und Sauerstoff enthält oder im wesentlichen aus diesen Gasen besteht. Dieser enthält üblicherweise noch Spuren von Feuchtigkeit. Üblicherweise wird daher eine Trocknungsstufe durchgeführt, in der der Gasstrom aus Chlor und Sauerstoff durch Inkontaktbringen mit geeigneten Trocknungsmitteln von Feuchtigkeitsspuren befreit wird. Geeignete Trocknungsmittel sind beispielsweise konzentrierte Schwefelsäure, Molsiebe oder hygroskopische Adsorbentien.

Schließlich wird aus dem getrockneten Gasstrom Chlor abgetrennt. Dabei wird auch ein Sauerstoff enthaltender Strom erhalten, der als Rückstrom in die Oxidationszone zurückgeführt werden kann. Die Chlor-Abtrennung erfolgt vorzugsweise durch Destillation, üblicherweise bei einer Temperatur im Bereich von -20 bis +50 °C und einem Druck im Bereich von 1 bis 20 bar in einer Destillationskolonne mit 10 bis 100 theoretischen Böden.

Es verbleibt ein im wesentlichen aus Chlor bestehender Strom, der gegenüber der eingesetzten ersten Teilmenge Chlor stark an Brom und Iod angereichert ist oder sogar im wesentlichen kein Brom und Iod mehr enthält. Dieser Chlorstrom wird zumindest teilweise als zweite Chlor-Teilmenge in Schritt (c) zurückgeführt.

Die Erfindung wird nachstehend unter Bezugnahme auf die Figuren näher erläutert.

Die Figur zeigt im Blockschema eine Ausführungsform des erfindungsgemäßen Verfahrens.

Ein erster Teilstrom Chlor 34, enthaltend weniger als 400 ppm Brom und Iod, ein zweiter Teilstrom Chlor 33 und ein Kohlenmonoxidstrom 35 werden der Phosgensynthesestufe 1 zugeführt und dort zu Phosgen umgesetzt, wobei Kohlenmonoxid bevorzugt im Überschuss eingesetzt wird. Der erhaltene Produktgasstrom 2, der im wesentlichen Phosgen und Kohlenmonoxid enthält und darüber hinaus noch Spuren von Chlor, Tetrachlorkohlenstoff und Inerte wie Stickstoff enthalten kann, wird der Trennstufe 3 zugeführt und dort, vorzugsweise durch Auskondensieren von Phosgen oder durch Destillation, in einen Abgasstrom 4, der im wesentlichen aus Kohlenmonoxid besteht gegebenenfalls Spuren von Chlor enthalten kann und einen Strom 5 aus Phosgen aufgetrennt. Der Kohlenmonoxidstrom 4 kann auch in die Phosgensynthese zurückgeführt werden. Der Phosgenstrom 5 enthält auch die in dem eingesetzten Chlor enthaltenen Anteile Brom und Iod. Diese können sowohl in molekularer Form als auch chemisch gebunden (z. B. als Bromphosgen) vorliegen. Dieser Strom 5, ein Strom 6 aus Amin, ein Phosgen-Rückstrom 17 und ein Lösemittel-Rückstrom 10 werden der Phosgenierungsstufe 7 zugeführt, wo die Umsetzung von Amin mit Phosgen zu Isocyanat und Chlorwasserstoff stattfindet. Die Phosgenierungsstufe 7 kann beispielsweise als Rührkessel, Rührkesselkaskade, Reaktionskolonne oder Rohrreaktor mit vorgeschaltetem Mischorgan oder Verschaltung der vorgenannten Apparate ausgestaltet sein. Die Phosgenierung kann zweistufig als Kaltphosgenierung mit anschließender Heißphosgenierung durchgeführt werden. Es wird ein flüssiger Produktstrom 8 enthaltend Lösemittel, Isocyanat und Nebenprodukte (z. B. Harnstoff, Oligomere) erhalten, aus dem in der nachfolgenden Trennstufe 9, vorzugsweise durch Destillation, das Lösemittel abgetrennt wird. Der Lösemittelstrom 10 wird unter Ersatz von Lösemittelverlusten in die Phosgenierungsstufe 7 zurückgeführt. Der verbleibende Isocyanatstrom 11 wird in der Reinigungsstufe 12 in Wertprodukt 13 und Schwersieder 14 aufgetrennt. Gegebenenfalls als Schwersieder anfallenden Oligomere können auch als Wertprodukt betrachtet werden. In der Phosgenierungsreaktion gebildeter Chlorwasserstoff und überschüssiges Phosgen verlassen die Phosgenierungsstufe 7 als Gasstrom 15, der auch Lösemittelreste, leichtsiedende Nebenprodukte, Kohlenmonoxid, Kohlendioxid sowie Inertgase (beispielsweise Stickstoff, Argon) enthalten kann. Aus diesem werden in der Trennstufe 16, vorzugsweise durch Destillation, Phosgen und Lösemittelreste abgetrennt und als Rückstrom 17 in die Phosgenierungsstufe 7 zurückgeführt. Es verbleibt ein Chlorwasserstoffstrom 18, der noch Spuren von Lösemittel, Phosgen oder Inerten enthalten kann. Dieser wird gegebenenfalls in einer Reinigungsstufe 19, vorzugsweise durch Absorption, von Lösemittelspuren befreit. Es wird ein gereinigter Chlorwasserstoffstrom 20 erhalten. Dieser und ein Sauerstoff enthaltender Strom 21, ein Sauerstoff enthaltender Rückstrom 31 und ein Chlorwasserstoff enthaltender Rückstrom 37 werden in einen Chlorwasserstoff-Oxidationsreaktor 22 eingespeist, in dem Chlorwasserstoff katalytisch zu Chlor oxidiert wird. Als Sauerstoff enthaltender Strom können beispielsweise reiner Sauerstoff, 94 vol.-%iger Sauerstoff aus einer Druckwechselabsorption (technisch reiner Sauerstoff) oder mit Sauerstoff angereicherte Luft eingesetzt werden. Es wird ein Produktgasstrom 23 erhalten, der im wesentlichen Chlor, nicht umgesetzten Sauerstoff, nicht umgesetzten Chlorwasserstoff und Wasserdampf enthält. Der Produktgasstrom 23 wird in einen Phasenkontaktapparat 24 eingeleitet und dort mit verdünnter Salzsäure 25 in Kontakt gebracht. Der mit dem abgetrennten Chlorwasserstoff beladene Strom 26 aus höher konzentrierter Salzsäure wird der Desorptionskolonne 36 zugeführt, in der der absorbierte Chlorwasserstoff wieder freigesetzt wird und als Rückstrom 37 dem Chlorwasserstoff-Oxidationsreaktor 22 zugeführt wird. Die bei der Desorption wiedergewonnene verdünnte Salzsäure wird gegebenenfalls gekühlt und teilweise als Strom 38 in den Phasenk-ontaktapparat 24 zurückgeführt. Den Phasenkontaktapparat 24 verlässt ein weitgehend von Chlorwasserstoff befreiter Strom 27 aus Chlor, Sauerstoff und Wasserdampf, der einer Trocknungsstufe 28 zugeleitet wird. In der Trocknungsstufe 28 wird der Gasstrom 27 mit einem geeigneten Absorptionsmittel wie Schwefelsäure, Molsiebe oder weitere hygroskopische Adsorbentien wie Silicagel oder Zeolithe in Kontakt gebracht und so von Wasserspuren befreit. Der getrocknete Gasstrom 29 aus Chlor und Sauerstoff wird der Abtrennstufe 30 zugeführt, in der Chlor vorzugsweise durch Kondensation abgetrennt wird. Es wird ein Sauerstoff enthaltender Strom erhalten, der auch beispielsweise bis zu 10 Vol.-% Chlor enthalten kann, und als Rückstrom 31 in den Chlorwasserstoff-Oxidationsreaktor zurückgeführt. Um die Aufpegelung von inerten Gasbestandteilen wie Stickstoff, Argon (gegebenenfalls aus dem Sauerstoff enthaltenden Strom 21, falls kein reiner Sauerstoff eingesetzt wird) oder Kohlendioxid (aus der Phosgenierung) zu vermeiden, ist ein Purge-Strom 32 vorgesehen. Aus der Abtrennstufe 30 wird weiterhin ein im wesentlichen Chlor enthaltender Strom 33 erhalten, welcher als zweiter Chlor-Teilstrom in die Phosgensynthesestufe 1 zurückgeführt wird.

## Patentansprüche

1. Verfahren zur Herstellung von organischen Isocyanaten mit den Schritten
(a) Bereitstellung einer ersten Teilmenge Chlor, wobei das Chlor der ersten Teilmenge einen Gehalt an freiem oder gebundenem Brom und Iod von < 400 ppm aufweist;
(b) Bereitstellung einer zweiten Teilmenge Chlor;
(c) Umsetzung der ersten und der zweiten Teilmenge Chlor mit Kohlenmonoxid zu Phosgen;
(d) Umsetzung des Phosgens aus Schritt (c) mit einem oder mehreren primären Aminen zu den entsprechenden Isocyanaten und Chlorwasserstoff;
(e) Abtrennung und gegebenenfalls Reinigung der in Schritt (d) gebildeten Isocyanate;
(f) Abtrennung und gegebenenfalls Reinigung des in Schritt (d) gebildeten Chlorwasserstoffs;
(g) katalytische Oxidation zumindest eines Teils des in Schritt (f) abgetrennten Chlorwasserstoffs mit Sauerstoff zu Chlor;
(h) Abtrennung des in Schritt (g) gebildeten Chlors und Einsatz zumindest einer Teilmenge des abgetrennten Chlors als zweite Teilmenge Chlor in Schritt (b).

2. Verfahren nach Anspruch 1, **dadurch gekennzeichnet, dass** das Chlor der ersten Teilmenge Chlor durch Elektrolyse einer Chloridionen enthaltenden Lösung erhalten wird.

3. Verfahren nach Anspruch 1 oder 2, **dadurch gekennzeichnet, dass** das Chlor der ersten Teilmenge in einer Abreicherungsstufe an Brom und/oder Iod abgereichert wird.

4. Verfahren nach einem der Ansprüche 1 bis 3, **dadurch gekennzeichnet, dass** im wesentlichen die Gesamtmenge des in Schritt (h) abgetrennten Chlors als zweite Teilmenge Chlor in Schritt (b) eingesetzt wird, und die erste Teilmenge Chlor so bemessen ist, dass die Summe aus erster und zweiter Teilmenge konstant bleibt.

5. Verfahren nach einem der Ansprüche 1 bis 4, **dadurch gekennzeichnet, dass** der Anteil der zweiten Teilmenge Chlor an der Summe aus erster und zweiter Teilmenge Chlor mindestens 70% beträgt.

6. Verfahren nach einem der Ansprüche 1 bis 5, **dadurch gekennzeichnet, dass** das Chlor der ersten Teilmenge einen Gehalt an freiem oder gebundenem Brom und Iod von < 100 ppm aufweist.

7. Verfahren nach einem der Ansprüche 1 bis 6, **dadurch gekennzeichnet, dass** die Chlorwasserstoff-Oxidation (g) heterogenkatalytisch durchgeführt wird.

8. Verfahren nach Anspruch 7, **dadurch gekennzeichnet, dass** die Chlorwasserstoffoxidation an einem Katalysator, enthaltend Rutheniumoxid auf einem Träger, ausgewählt aus der Gruppe bestehend aus Siliziumdioxid, Aluminiumoxid, Titandioxid, Zirkondioxid und deren Gemischen, durchgeführt wird.

9. Verfahren nach einem der Ansprüche 1 bis 8, **dadurch gekennzeichnet, dass** das oder die primären Amine ausgewählt sind aus der Gruppe bestehend aus den isomeren, monomeren und oligomeren Diphenylmethandiaminen, isomeren Toluylendiaminen, Isophorondiamin und Hexamethylendiamin.

10. Verfahren nach einem der Ansprüche 1 bis 10, **dadurch gekennzeichnet, dass** die Chlorwasserstoff-Oxidation in einem Festbett- oder Wirbelbettreaktor durchgeführt wird.

## Claims

1. A process for preparing organic isocyanates, which comprises the steps
(a) making available a first partial amount of chlorine, with the chlorine of this first partial amount having a content of free and bound bromine and iodine of <400 ppm;
(b) making available a second partial amount of chlorine;
(c) reacting the first and second partial amounts of chlorine with carbon monoxide to form phosgene;
(d) reacting the phosgene from step (c) with one or more primary amines to form the corresponding isocyanates and hydrogen chloride;
(e) separating off and, if necessary, purifying the isocyanates formed in step (d);
(f) separating off and, if necessary, purifying the hydrogen chloride formed in step (d);
(g) catalytically oxidizing at least part of the hydrogen chloride separated off in step (f) by means of oxygen to form chlorine;
(h) separating off the chlorine formed in step (g) and using at least a partial amount of the chlorine which has been separated off as second partial amount of chlorine in step (b).

2. The process according to claim 1, wherein the chlorine of the first partial amount of chlorine is obtained by electrolysis of a solution comprising chloride ions.

3. The process according to claim 1 or 2, wherein the chlorine of the first partial amount is depleted in bromine and/or iodine in a purification stage.

4. The process according to any of claims 1 to 3, wherein essentially the total amount of the chlorine separated off in step (h) is used as second partial amount of chlorine in step (b), and the first partial amount of chlorine is calculated so that the sum of first and second partial amounts remains constant.

5. The process according to any of claims 1 to 4, wherein the second partial amount of chlorine makes up at least 70% of the sum of first and second partial amounts of chlorine.

6. The process according to any of claims 1 to 5, wherein the chlorine of the first partial amount has a content of free and bound bromine and iodine of <100 ppm.

7. The process according to any of claims 1 to 6, wherein the hydrogen chloride oxidation (g) is carried out in the presence of a heterogeneous catalyst.

8. The process according to claim 7, wherein the hydrogen chloride oxidation is carried out over a catalyst comprising ruthenium oxide on a support selected from the group consisting of silicon dioxide, aluminum oxide, titanium dioxide, zirconium dioxide and mixtures thereof.

9. The process according to any of claims 1 to 8, wherein the primary amine or amines is/are selected from the group consisting of the isomeric, monomeric and oligomeric diphenylmethanediamines, isomeric toluenediamines, isophoronediamine and hexamethylenediamine.

10. The process according to any of claims 1 to 10, wherein the hydrogen chloride oxidation is carried out in a fixed-bed or fluidized-bed reactor.

## Revendications

1. Procédé de fabrication d'isocyanates organiques, comprenant les étapes
(a) mise à disposition d'un premier sous-ensemble de chlore, le chlore du premier sous-ensemble présentant une teneur en brome et iode libres ou reliés < 400 ppm ;
(b) mise à disposition d'un second sous-ensemble de chlore ;
(c) mise en réaction du premier et du second sous-ensemble de chlore avec du monoxyde de carbone pour former du phosgène ;
(d) mise en réaction du phosgène de l'étape (c) avec une ou plusieurs amines primaires pour former les isocyanates correspondants et du chlorure d'hydrogène ;
(e) séparation et éventuellement purification des isocyanates formés à l'étape (d) ;
(f) séparation et éventuellement purification du chlorure d'hydrogène formé à l'étape (d) ;
(g) oxydation catalytique d'au moins une partie du chlorure d'hydrogène séparé à l'étape (f) avec de l'oxygène pour former du chlore ;
(h) séparation du chlore formé à l'étape (g) et utilisation d'au moins un sous-ensemble du chlore séparé en tant que second sous-ensemble de chlore à l'étape (b).

2. Procédé selon la revendication 1, **caractérisé en ce que** le chlore du premier sous-ensemble de chlore est obtenu par électrolyse d'une solution contenant des ions chlorure.

3. Procédé selon la revendication 1 ou 2, **caractérisé en ce que** le chlore du premier sous-ensemble est appauvri en brome et/ou en iode lors d'une étape d'appauvrissement.

4. Procédé selon l'une quelconque des revendications 1 à 3, **caractérisé en ce qu'**essentiellement la totalité du chlore séparé à l'étape (h) est utilisée en tant que second sous-ensemble de chlore à l'étape (b), et le premier sous-ensemble de chlore est mesuré de manière à ce que la somme du premier et du second sous-ensemble reste constante.

5. Procédé selon l'une quelconque des revendications 1 à 4, **caractérisé en ce que** la proportion du second sous-ensemble de chlore par rapport à la somme du premier et du second sous-ensemble de chlore est d'au moins 70 %.

6. Procédé selon l'une quelconque des revendications 1 à 5, **caractérisé en ce que** le chlore du premier sous-ensemble présente une teneur en brome et iode libres ou reliés < 100 ppm.

7. Procédé selon l'une quelconque des revendications 1 à 6, **caractérisé en ce que** l'oxydation du chlorure d'hydrogène (g) est réalisée sous catalyse hétérogène.

8. Procédé selon la revendication 7, **caractérisé en ce que** l'oxydation du chlorure d'hydrogène est réalisée sur un catalyseur qui contient de l'oxyde de ruthénium sur un support, choisi dans le groupe constitué du dioxyde de silicium, de l'oxyde d'aluminium, du dioxyde de titane, du dioxyde de zirconium et leurs mélanges.

9. Procédé selon l'une quelconque des revendications 1 à 8, **caractérisé en ce que** la ou les amines primaires sont choisies dans le groupe constitué des diphénylméthane diamines isomères, monomères et oligomères, des toluylène diamines isomères, de l'isophorone diamine et de l'hexaméthylène diamine.

10. Procédé selon l'une quelconque des revendications 1 à 10, **caractérisé en ce que** l'oxydation du chlorure d'hydrogène est réalisée dans un réacteur à lit fixe ou à lit fluidisé.
